# EUROPÄISCHE PATENTSCHRIFT

(11) **EP 1 876 230 B1**
(45) Veröffentlichungstag und Bekanntmachung des Hinweises auf die Patenterteilung: **17.09.2008**
(21) Anmeldenummer: 07107776.2
(22) Anmeldetag: 09.05.2007
(51) Int. Cl.: C12M 1/26

(54) **Probenahmeventil zur sterilen Entnahme von Proben aus einem Behälter oder einem Rohrleitungssystem**
Sample extractor valve for sterile extraction of samples from a vessel or a piping system
Vanne de prélèvement d'échantillon destinée au prélèvement stérile d'échantillon d'un récipient ou d'un système de conduite

(30) Priorität: 07.07.2006 DE 102006031840
(43) Veröffentlichungstag der Anmeldung: 09.01.2008
(73) Patentinhaber: VIEGA GmbH & Co. KG, 57439 Attendorn (DE)
(72) Erfinder: Wasserhövel, Thorsten, 59757, Arnsberg (DE)
(74) Vertreter: Cohausz & Florack

(56) Entgegenhaltungen:
- DE-U1- 20 316 936
- FR-A1- 2 617 286
- RU-C- 2 257 471

## Beschreibung

Die Erfindung betrifft ein Probenahmeventil zur sterilen Entnahme von Proben aus einem Behälter oder einem Rohrleitungssystem mit einem Ventilgehäuse mit einem Ventileinlass, einem Ventilauslass und einem diese verbindenden Strömungskanal, wobei der Ventileinlass mit einer ein entnehmbares Medium führenden Rohrleitung in Fluidverbindung stehen kann und wobei der Ventilauslass mit einem Ablassrohr in Fluidverbindung stehen kann, mit einem im Ventilgehäuse hin- und herbewegbaren, den Strömungskanal wahlweise in einer Schließstellung verschließenden und in einer Offenstellung freigebenden Ventilstößel und mit einer im Ventilgehäuse beweglich geführten und mit dem Ventilstößel verbundenen Betätigungswelle, wobei eine Bewegung der Betätigungswelle auf den Ventilstößel übertragbar ist, um diesen zwischen der Schließstellung und der Offenstellung zu bewegen. Ferner betrifft die Erfindung einen Ventileinsatz zum lösbaren Verbinden mit einem Ventilgehäuse, insbesondere einem Ventilgehäuse eines Probenahmeventils.

Probenahmeventile werden eingesetzt, um zu vorbestimmten Zeitpunkten Proben aus einem Behälter, beispielsweise einem Fermenter oder Bioreaktor, oder aus einem Rohrleitungssystem, beispielsweise einem Trinkwassersystem, insbesondere einem Trinkwassersystem in Krankenhäusern, zur späteren Analyse zu entnehmen. Bei Fermentern oder Bioreaktoren kann mit einer solchen Analyse ein Fortschritt oder Ablauf eines biologischen oder technologischen Reaktionsprozesses verfolgt werden. Bei Trinkwassersystemen dient die Analyse insbesondere zur Bestimmung der mikrobiologischen und chemischen Parameter, die durch die Trinkwasserverordnung festgesetzt sind. Letzteres ist insbesondere in Krankenhäusern von Bedeutung.

Die Probenahme unterliegt bestimmten Sterilitätsbedingungen, denen auch das Probenahmeventil genügen muss. Das Probenahmeventil ist dabei hochgradig steril und muss insbesondere möglichst frei von Keimen, Bakterien, Verschmutzungen etc. sein. Um die geforderte Sterilität zu gewährleisten, ist es beispielsweise aus der DE 203 16 936 U1 bekannt, ein Probenahmeventil der zuvor beschriebenen Art regelmäßig einem Sterilisationsprozess zu unterziehen, bei dem das Ventil von einem Sterilisationsmittel, beispielsweise Wasserdampf, durchströmt wird. Da die Probe bei der Entnahme aber nicht erhitzt werden darf, da sie sonst unbrauchbar werden kann, insbesondere bei zu untersuchenden Proteinen, die bei starker Erwärmung denaturieren können, ist außerdem eine Kühleinrichtung integriert, die das nach dem Dampfspülen erhitzte Ventil vor der nächsten Probenahme abkühlt.

Das Probenahmeventil aus dem Stand der Technik ist aufgrund seiner Vielzahl an Bauteilen relativ aufwendig und damit teuer in der Herstellung. Außerdem ist die Handhabung beim Sterilisierung und Abkühlen relativ kompliziert. Schließlich kann aufgrund der vielen Bauteile und der entsprechend großen Anzahl an Kanten, Ecken und Toträumen auf lange Sicht ein gewisser Grad an Keimbildung nicht völlig ausgeschlossen werden. Das Ventil muss daher zwangsläufig nach mehreren Probenahmen vollständig demontiert und einer grundlegenden Reinigung unterzogen werden, was beides einen hohen Aufwand bedeutet. Alternativ können auch die maßgeblichen Teile des Probenahmenventils nach der aufwendigen Demontage durch neue Bauteile ersetzt werden, was aber relativ hohe Kosten verursacht.

Es ist daher die Aufgabe der vorliegenden Erfindung, das eingangs genannte Probenahmeventil so weiterzuentwickeln, dass die Hygieneeigenschaften verbessert werden.

Die zuvor hergeleitete und aufgezeigte Aufgabe wird gemäß einer ersten Lehre der vorliegenden Erfindung bei einem Probenahmeventil der eingangs genannten Art dadurch gelöst, dass der Ventilstößel in einem lösbar mit dem Ventilgehäuse verbundenen Adapterkörper beweglich gelagert ist.

Dadurch, dass ein Adapterkörper vorgesehen ist, welcher zur Lagerung des Ventilstößels dient und welcher lösbar mit dem Ventilgehäuse verbunden ist, wird eine einfache Demontage des Probenahmeventils ermöglicht. Auf diese Weise kann in regelmäßigen Abständen ohne großen Aufwand eine Grundreinigung zum Entfernen evtl. angesammelter Keime, Bakterien, Verschmutzungen etc. durchgeführt werden. Dazu ist es lediglich erforderlich, die Rohrleitung am Ventileinlass zu lösen und mit einem Handgriff den Adapterkörper mit innenliegendem Ventilstößel abzunehmen. Dieser kann dann, beispielsweise durch Wasserdampf oder ein Reinigungsbad, sterilisiert werden. Im ausgebauten Zustand sind die einzelnen Bauteile auch wesentlich einfacher zu reinigen, als im montierten Zustand. Entsprechend kann auch das Ventilgehäuse auf einfache Weise, beispielsweise mit Wasserdampf, durchspült werden, insbesondere dann, wenn auch die Betätigungswelle abgenommen wurde.

Aufgrund der einfachen Montage und Demontage ist der Austausch einzelner Bauteile zu Wartungs- und Reparaturzwecken ebenfalls relativ einfach. Außerdem können einzelne Bauteile, beispielsweise der Ventileinsatz mit Adapterkörper und innenliegendem Ventilstößel, auch kurzerhand in anderen Anlagen eingesetzt werden oder an den jeweiligen Probenahmestellen verbleiben, wobei dann das Ventilgehäuse mobil einsetzbar ist. Ferner ist es möglich, in regelmäßigen Abständen, beispielsweise sogar für jede Probenahme erneut, einen bereits zuvor gereinigten Ventileinsatz einzusetzen. Es ist dann nicht mehr erforderlich, unmittelbar vor der Probenahme das Probenahmeventil zu sterilisieren und abzuwarten, bis dieses abgekühlt ist.

Gemäß einer vorteilhaften Ausgestaltung des Probenahmeventils weist der Ventilstößel einen Stößelabschnitt auf, der mit einem am Adapterkörper ausgebildeten Ventilsitz und/oder einer am Adapterkörper ausgebildeten Dichtfläche dichtend zusammenwirkt. Insbesondere bei Verwendung eines kegelförmigen Ventilsitzes in Verbindung mit einer zylinderförmigen Dichtfläche wird eine gute Dichtwirkung in der Schließstellung erzielt. Dabei kann der Stößelabschnitt, der vorzugsweise mindestens einen Ventilteller aufweist, gegenüber dem Adapterkörper, insbesondere der Dichtfläche oder dem Ventilsitz, durch ein Dichtelement, insbesondere einen O-Ring, abgedichtet sein. Dies erhöht die Dichtwirkung zusätzlich.

Gemäß einer weiteren vorteilhaften Ausgestaltung ist der Adapterkörper mit dem Ventilgehäuse durch eine Rast-, Klemm- und/oder Steckverbindung, insbesondere gesicherte Steckverbindung, verbunden. Auf diese Weise läßt sich der Adapterkörper einfach und mit einem Handgriff oder zumindest mit nur einer Hand vom Ventilgehäuse lösen. Insbesondere die Rast- und Steckverbindung läßt außerdem zu, dass der Adapterkörper im Ventilgehäuse drehbar, insbesondere um 360°, angeordnet ist, was eine Vielzahl von Anschlussmöglichkeiten zuläßt, wenn das Probenahmeventil mit einer bestehenden Anlage verbunden werden soll. Es ist auch denkbar, den Adapterkörper über eine Schraubverbindung mit dem Ventilgehäuse zu verbinden, da sich eine Schraubverbindung ebenfalls relativ einfach mit wenigen Handgriffen lösen läßt.

Der Adapterkörper ist gemäß einer weiteren vorteilhaften Ausgestaltung gegenüber dem Ventilgehäuse durch mindestens ein Dichtelement, insbesondere einen O-Ring, abgedichtet. Eine besonders gute Dichtwirkung wird erzielt, wenn mehrere Dichtelemente verwendet werden, von denen eines zumindest ein O-Ring ist.

Da der Adapterkörper gegenüber dem Ventilgehäuse gut abgedichtet ist, kann gemäß noch einer weiteren vorteilhafte Ausgestaltung der Adapterkörper einen Anschluss zur Verbindung mit der Rohrleitung aufweisen. Die Rohrleitung muss in diesem Fall nicht unmittelbar an das Ventilgehäuse angeschlossen werden, sondern ist, beispielsweise über ein Schraubverbindung, fest mit dem Ventileinsatz verbunden. Dies ermöglicht beispielsweise auch eine Demontage des Probenahmeventils, ohne dass vorher die Rohrleitung vom Ventileinlass abgenommen werden muss, da der Ventileinsatz bzw. der Adapterkörper lösbar mit dem übrigen Probenahmeventil verbunden ist. Wird das Probenahmeventil beispielsweise zu Reinigungszwecken vom Ventileinsatz getrennt, so verbleibt der Ventileinsatz am Ende der Rohrleitung und verschließt diese automatisch.

Gemäß einer anderen vorteilhaften Ausgestaltung des erfindungsgemäßen Probenahmeventils ist der Adapterkörper mit mindestens einer, vorzugsweise drei, Entleerungsbohrungen versehen. Bei Verwendung von drei Entleerungsbohrungen sind diese vorzugsweise in einem Winkel von 120° über den Umfang des Adapterkörpers verteilt angeordnet. Dabei kann die eine oder die mehreren Entleerungsbohrungen in Strömungsrichtung hinter dem Ventilsitz oder der Dichtfläche liegen. Auf diese Weise kann zu Reinigungszwecken der Ventileinsatz vom übrigen Probenahmeventil getrennt werden, wobei auch letzte Reste des Probemediums ohne weiteres abfließen. Auch eine Reinigung ist auf diese Weise deutlich einfacher, da das Reinigungsmittel einfacher abfließt und Adapterkörper und Ventilstößel schneller trocknen. Es wird ein Probenahmeventil geschaffen, welches totraumfrei ausgebildet ist, bei dem also jeder Raum durchspülbar ist.

Gemäß noch einer anderen vorteilhaften Ausgestaltung dringt das mit dem Ventilstößel verbundene Ende der Betätigungswelle, die beispielsweise über einen Inbus bedienbar ist, in Offenstellung in das Innere des Adapterkörpers ein. Dies ermöglicht eine kompakte Bauweise des Ventilgehäuses und damit des Probenahmeventils, da hier im Betrieb relativ zueinander bewegliche Bauteile überlappen können.

Ein besonders einfacher Aufbau wird dadurch erreicht, dass gemäß einer weiteren vorteilhaften Ausgestaltung der Adapterkörper, der Ventilstößel und/oder die Betätigungswelle koaxial zueinander angeordnet sind. Die koaxiale Anordnung führt einerseits zu einer einfachen Herstellung des Ventilgehäuses, ermöglicht aber auch eine besonders einfache Montage oder Demontage der einzelnen Bauteile. Auch das Reinigen des Ventilgehäuses wird damit optimiert, da das Ventilgehäuse auf einfache Weise durchspült werden kann und ein Minimum an Ecken und Kanten aufweist.

Gemäß einer vorteilhaften Ausgestaltung des erfindungsgemäßen Probenahmeventils ist die Bewegung der Betätigungswelle translatorisch und/oder rotatorisch. Auch die Bewegung des Ventilstößels kann translatorisch und/oder rotatorisch sein. Insbesondere bei einem unter relativ hohem Druck stehenden Medium ist es vorteilhaft, wenn die Betätigungswelle als Spindel ausgebildet ist und insbesondere in einem im Ventilgehäuse ausgebildeten Innengewinde geführt ist. Auf diese Weise kann, beispielsweise über einen Inbus, eine rotatorische und gleichzeitig translatorische Bewegung auf die Betätigungswelle übertragen werden, wobei sich zumindest die translatorische Bewegung auch auf den Ventilstößel überträgt.

Gemäß einer weiteren vorteilhaften Ausgestaltung sind die Betätigungswelle und der Ventilstößel lose miteinander verbunden und stehen insbesondere in Flächenkontakt miteinander. Dies ermöglicht eine besonders einfache Demontage, da lediglich der Ventileinsatz durch beispielsweise eine Steckverbindung mit dem Ventilgehäuse verbunden ist. Wird das Ventilgehäuse vom Adapterkörper getrennt, löst sich auch gleichzeitig die Verbindung zwischen Ventilstößel und Betätigungswelle. Dabei kann zur Erhöhung der Stabilität die Betätigungswelle eine Aufnahme für ein Ende des Ventilstößels haben. Auf diese Weise ist der Ventilstößel auch zusätzlich gelagert.

Gemäß einer anderen vorteilhaften Ausgestaltung des erfindungsgemäßen Probenahmeventils ist der Ventilstößel in Richtung der Schließstellung mit Federkraft beaufschlagt. Dies ist insbesondere dann von Vorteil, wenn der Ventilstößel und die Betätigungswelle nur lose miteinander verbunden sind. Auf diese Weise kann die Betätigungswelle den Ventilstößel aktiv in Offenstellung bewegen, wobei beim Zurückbewegen der Betätigungswelle der Ventilstößel unter anderem durch die Federkraft zurück in die Schließstellung gedrückt wird. Neben der Federkraft wirkt aber auch der Druck des Mediums auf den Ventilstößel in Richtung der Schließstellung. Um die Federkraft auf den Ventilstößel zu übertragen, ist insbesondere eine Schraubenfeder im Innern des Adapterkörpers angeordnet.

Bei dem erfindungsgemäßen Probenahmeventil ist gemäß einer anderen vorteilhaften Ausgestaltung vorgesehen, dass das Ventilgehäuse T-förmig ausgebildet ist, wobei der Strömungskanal am Ventilauslass in einem Winkel von inbesondere 90° zum Strömungskanal am Ventileinlass angeordnet ist. Die T-förmige Ausbildung ermöglicht, wie zuvor dargestellt, eine besonders effektive Reinigung und Sterilisierung des Probenahmeventils. Auch der Aufwand bei der Herstellung des Ventilgehäuses kann dadurch reduziert werden.

Wie bereits zuvor beschrieben, kann gemäß einer vorteilhaften Ausgestaltung der Adapterkörper im Ventilgehäuse drehbar, insbesondere um 360°, angeordnet sein. Um die Freihheitsgrade beim Anschluss an eine bestehende Anlage weiter zu erhöhen, kann gemäß noch einer weiteren vorteilhaften Ausgestaltung auch das Ablassrohr relativ zum Ventilgehäuse drehbar, insbesondere um 360°, angeordnet sein. Dazu ist das Ablassrohr mit dem Ventilgehäuse vorzugsweise über einen Klemmring und eine Druckschraube verbunden. Damit wird einerseits eine dichte Verbindung geschaffen, die andererseits aber auch die Drehbarkeit zuläßt.

Das Ventilgehäuse ist gemäß wiederum einer weiteren Ausgestaltung des Probenahmeventils aus Rotguss und/oder Edelstahl gefertigt. Alternativ oder zusätzlich kann auch der Adapterkörper, der Ventilstößel und/oder die Betätigungswelle aus Rotguss und/oder Edelstahl gefertigt sein. Rotguss hat gute Gleiteigenschaften, ist veschleißfest und weist eine relativ hohe Zugfestigkeit auf. Daher sind Rotgusslegierungen gerade bei den beweglichen Teilen im erfindungsgemäßen Probenahmeventil besonders vorteilhaft. Entsprechendes gilt auch für Edelstahl, was sich aus durch eine besonders gute Korrosionsbeständigkeit auszeichnet.

Die zuvor hergeleitete und aufgezeigte Aufgabe wird ferner gemäß einer zweiten Lehre der vorliegenden Erfindung gelöst durch einen Ventileinsatz zum lösbaren Verbinden mit einem Ventilgehäuse, insbesondere einem Ventilgehäuse eines Probenahmeventils, mit einem im Ventilgehäuse hin- und herbewegbaren, einen Strömungskanal im Ventilgehäuse wahlweise in einer Schließstellung verschließenden und in einer Offenstellung freigebenden Ventilstößel und mit einem Adapterkörper, in dem der Ventilstößel beweglich gelagert ist.

Ein solcher Ventileinsatz, dessen Bestandteile, insbesondere dessen Adapterkörper und Ventilstößel, wie zuvor beschrieben ausgebildet sein können, hat neben den genannten Vorzügen auch den Vorteil, dass dieser ohne weiteres in verschiedenen Ventileinrichtungen eingesetzt werden kann. So ist ein solcher Ventileinsatz in jedweder Form von Ventil, insbesondere Probenahmeventil, einsetzbar, auch nachträglich, und führt zu deutlich verbesserten Hygieneeigenschaften.

Es gibt nun eine Vielzahl von Möglichkeiten, das erfindungsgemäße Probenahmeventil und den erfindungsgemäßen Ventileinsatz auszugestalten und weiterzubilden. Hierzu wird beispielsweise verwiesen einerseits auf die abhängigen Patentansprüche, andererseits auf die Beschreibung eines Ausführungsbeispiels in Verbindung mit der Zeichnung. In der Zeichnung zeigt:
- Fig. 1: eine Schnittansicht eines Ausführungsbeispiels eines Probenahmeventils in Schließstellung,
- Fig. 2: eine Schnittansicht des Probenahmeventils aus Fig. 1 in Offenstellung,
- Fig. 3: eine Schnittansicht des Probenahmeventils aus Fig. 1 mit demontiertem Ventileinsatz und
- Fig. 4: eine Schnittansicht eines Ausführungsbeispiels einer Probenahmestelle in einem Rohrleitungssystem.

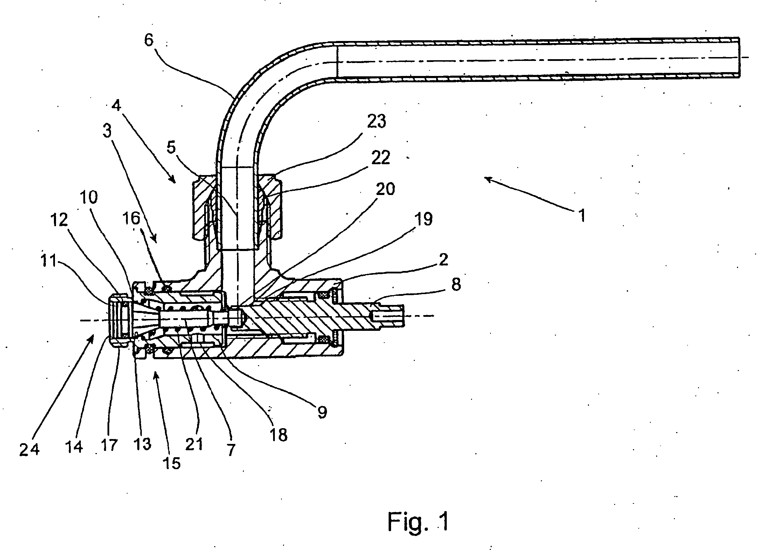

In Fig. 1 ist ein Probenahmeventil 1 zur sterilen Entnahme von Proben aus einem Behälter oder einem Rohrleitungssystem dargestellt. Das Probenahmeventil 1 weist ein Ventilgehäuse 2 mit einem Ventileinlass 3, einem Ventilauslass 4 und einem diese verbindenden Strömungskanal 5 auf, wobei der Ventileinlass 3 mit einem Adapterkörper 9 verbunden ist, der wiederum mit einer ein entnehmbares Medium führenden (nicht dargestellten) Rohrleitung verbindbar ist und wobei der Ventilauslass 4 mit einem Ablassrohr 6 verbunden ist.

Im Adapterkörper 9 ist ein Ventilstößel 7 beweglich geführt, so dass der Strömungskanal 5 wahlweise in einer Schließstellung verschlossen und in einer Offenstellung freigegeben werden kann. In Fig. 1 ist die Schließstellung dargestellt.

Ferner ist eine Betätigungswelle 8 im Ventilgehäuse 2 beweglich geführt und mit dem Ventilstößel 7 lose verbunden. Eine Bewegung der Betätigungswelle 8 ist damit auf den Ventilstößel 7 übertragbar, um diesen zwischen der Schließstellung und der Offenstellung zu bewegen.

Der Ventilstößel 7 ist bei dem erfindungsgemäßen Probenahmeventil 1 in dem Adapterkörper 9 beweglich gelagert, wobei der Adapterkörper 9 lösbar mit dem Ventilgehäuse 2 verbunden ist.

Der Ventilstößel 7 ist im dargestellten Ausführungsbeispiel mit einem Stößelabschnitt 10, der zwei Ventilteller 11 und ein dazwischenliegendes als O-Ring ausgebildetes Dichtelement 12 aufweist, versehen. Ferner ist ein Teil des Stößelabschnitts 10 im Querschnitt V-förmig ausgebildet. Auf diese Weise wird zwischen Ventilstößel 7 und Adapterkörper 9 eine zweifache Dichtung erreicht, nämlich zum einen zwischen dem V-förmigen Abschnitt und einem kegelförmigen Ventilsitz 13 und zum anderen zwischen dem Dichtelement 12 bzw. den Ventiltellern 11 und einer zylindrischen Dichtfläche 14 am Adapterkörper 9.

Der Adapterkörper 9 ist gegenüber dem Ventilgehäuse 2 im vorliegenden Fall durch eine gesicherte Steckverbindung 15 verbunden, wobei die Steckverbindung durch eine Sicherungsfeder 24 gegen herausziehen gesichert wird.

Der Adapterkörper 9 ist ferner mit einem Anschluss 17 zur Verbindung mit der nicht dargestellten Rohrleitung versehen.

Am gegenüberliegenden Ende weist der Adapterkörper 9 drei Entleerungsbohrungen 18 auf, die über den Umfang des Adapterkörpers in einem Winkel von jeweils 120° angeordnet sind und die in Strömungsrichtung hinter dem Ventilsitz 13 und der Dichtfläche 12 liegen. Die Entleerungsbohrungen 18 erlauben ein schnelles und ungehindertes Abfließen evtl. im Adapterkörper 9 befindlicher Restmengen, sei es des Probemediums oder eines Reinigungsmittels wie Wasserdampf.

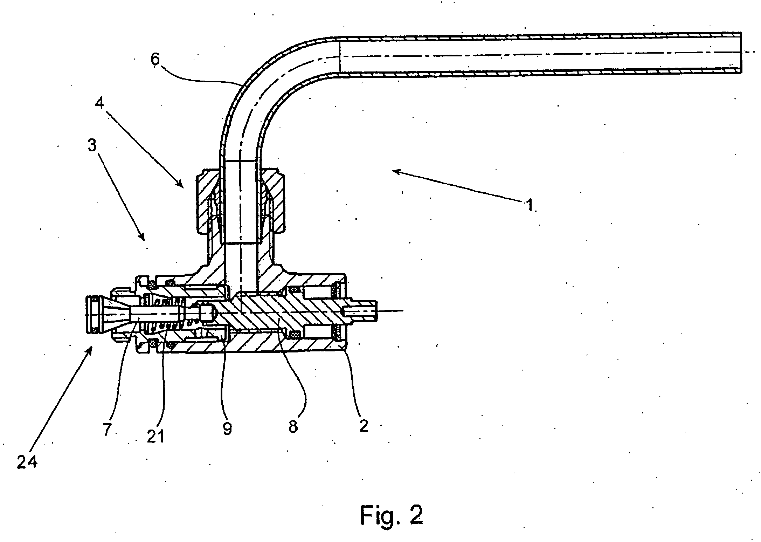

Eine weitere Besonderheit des vorliegenden Probenahmeventils 1 ist, dass, wie in Fig. 2 dargestellt, der Adapterkörper 9 im wesentlichen als Hohlzylinder ausgebildet ist, wobei der Innendurchmesser so gewählt ist, dass das mit dem Ventilstößel 7 verbundene Ende der Betätigungswelle 8 in Offenstellung, also bei geöffnetem Ventil, in das Innere des Adapterkörpers 9 eindringt.

Beim Vergleich der Fig. 1 (Schließstellung) und Fig. 2 (Offenstellung) ist ferner zu erkennen, dass die Bewegung der Betätigungswelle 8 sowohl translatorisch als auch rotatorisch ist, um den Ventilstößel 7 aus dem Adapterkörper 9 herauszubewegen. Die gleichzeitig translatorische und rotatorische Bewegung der Betätigungswelle 8 wird dadurch erreicht, dass die Betätigungswelle 8 als Spindel ausgebildet ist und in einem im Ventilgehäuse 2 ausgebildeten Innengewinde 19 geführt ist. Dabei wird die rotatorische Bewegung der spindelförmigen Betätigungswelle 8 durch einen Mehrkant (nicht dargestellt) übertragen.

Ein weiteres Kennzeichen des vorliegenden Ausführungsbeispiels eines Probenahmeventils 1 ist, dass die Betätigungswelle 8 eine Aufnahme 20 für ein Ende des Ventilstößels 7 aufweist, wobei die Aufnahme die Stabilität des koaxial zur Betätigungswelle 8 angeordneten Ventilstößels 7 erhöht, da der Ventilstößel 7 auf diese Weise an seinen beiden Enden gelagert ist, das heißt am linken Ende im Adapterkörper 9 und am rechten Ende in besagter Aufnahme 20.

Damit der Ventilstößel 7 aus der in Fig. 2 dargestellten Offenstellung beim Zurückbewegen der Betätigungswelle 8 ebenfalls in seiner Ausgangsstellung, das heißt die Schließstellung, zurückgeführt wird, ist eine Schraubenfeder 21 im Innern des Adapterkörpers 9 angeordnet, die der Ventilstößel 7 in Richtung der Schließstellung mit Federkraft beaufschlagt. Neben der Federkraft wirkt auch der Druck des strömenden Mediums auf den Ventilstößel 7 und drückt diesen in Richtung seiner Schließstellung.

Noch eine Besonderheit des dargestellten Ausführungsbeispiels ist, dass das Ventilgehäuse 2, welches T-förmig ausgebildet ist, relativ zum Adapterkörper 9 um die Längsachse des Adapterkörpers 9 um 360° drehbar ist. Ferner ist auch das Ablassrohr 6 relativ zum Ventilgehäuse 2 um 360° drehbar. Dazu ist das Ablassrohr 6 mit dem Ventilgehäuse 2 über einen Klemmring 22 und eine Druckschraube 23 verbunden. Auf diese Weise ist das erfindungsgemäße Probenahmeventil 1 in eine Vielzahl unterschiedlich aufgebauter Anlagen in nahezu jeder denkbaren Position montierbar.

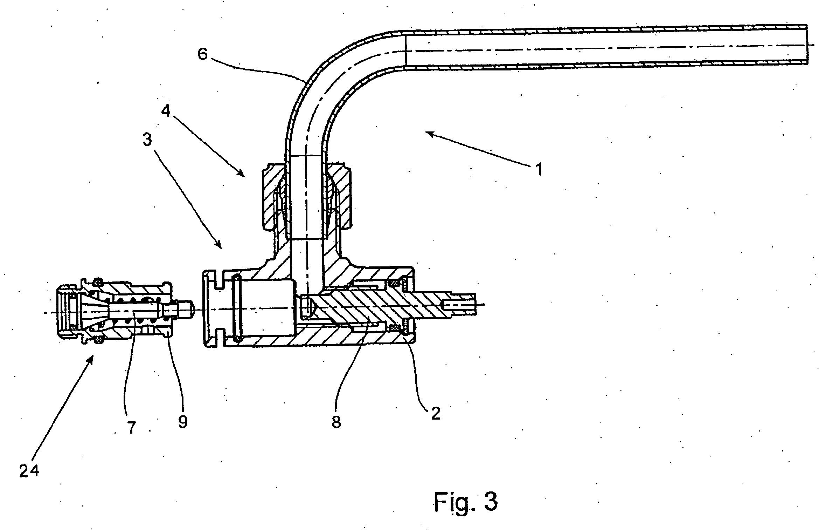

Fig. 3 zeigt das zuvor beschriebene Probenahmeventil 1 in teilweise demontiertem Zustand. Dabei ist die Steckverbindung zwischen Adapterkörper 9 und Ventilgehäuse 2 nach dem Entsichern durch bloßes Auseinanderziehen gelöst worden. Der Adapterkörper 9 mit innenliegendem Ventilstößel 7, der Schraubenfeder 21 und den beiden Dichtelementen 12 und 16 stellt einen gut zu reinigenden und einfach demontierbaren Ventileinsatz dar. Dabei kann der Ventileinsatz in dem in Fig. 1 dargestellten Zustand mit seinem Anschluss 17 nach wie vor an der nicht dargestellten Rohrleitung befestigt sein, was seine Handhabung ebenfalls deutlich vereinfacht.

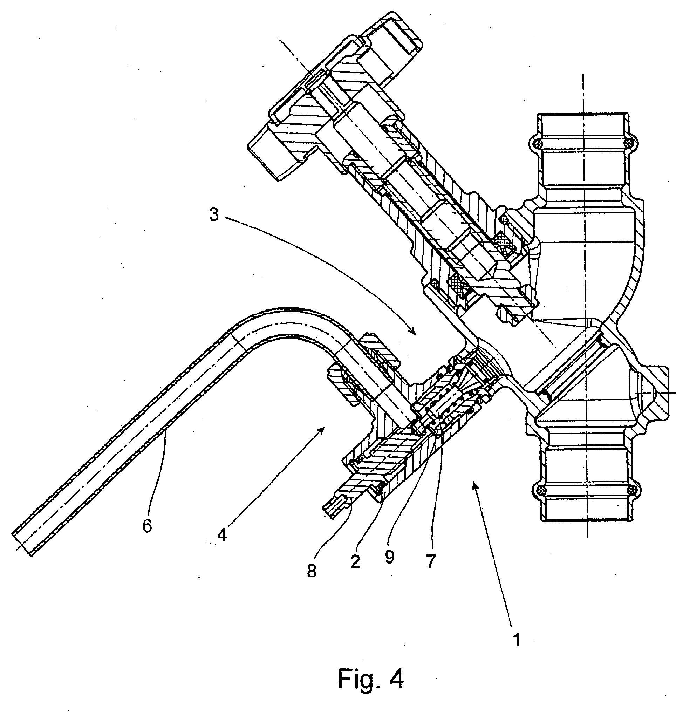

Fig. 4 zeigt schließlich einen Teil eines Rohrleitungssystems, beispielsweise eines Trinkwassersystems, bei dem ein Probenahmeventil, wie es zuvor anhand der Figuren 1 bis 3 beschrieben wurde, angeordnet ist. Dabei ist der Adapterkörper 9 über den Anschluss 17 mit einer Armatur durch Verschrauben fest verbunden. Der übrige Teil des Probenahmeventils 1 ist dabei, wie zuvor beschrieben, mittels Steckverbindung auf den Adapterkörper 9 aufgesteckt und kann auf diese Weise zu Reinigungszwecken mit einer Hand gelöst werden.

Das zuvor anhand der Figuren 1 bis 4 beschriebene Probenahmeventil 1 ist einfach aufgebaut und, da jeder Raum durchspülbar ist, nahezu totraumfrei. Außerdem ist das Probenahmeventil mit einem einzigen Handgriff vom Ventileinsatz 24 trennbar, was sowohl die Reinigung als auch den Austausch einzelner Bauteile zu Wartungs- und Reparaturzwecken besonders einfach gestaltet. Schließlich ist das erfindungsgemäße Probenahmeventil auch aufgrund seines einfachen Aufbaus und seiner einfachen Handhabung aus hygienischer Sicht vorteilhaft.

## Patentansprüche

1. Probenahmeventil (1) zur sterilen Entnahme von Proben aus einem Behälter oder einem Rohrleitungssystem
- mit einem Ventilgehäuse (2) mit einem Ventileinlass (3), einem Ventilauslass (4) und einem diese verbindenden Strömungskanal (5), wobei der Ventileinlass (3) mit einer ein entnehmbares Medium führenden Rohrleitung in Fluidverbindung stehen kann und wobei der Ventilauslass (4) mit einem Ablassrohr (6) in Fluidverbindung stehen kann,
- mit einem im Ventilgehäuse (2) hin- und herbewegbaren, den Strömungskanal (5) wahlweise in einer Schließstellung verschließenden und in einer Offenstellung freigebenden Ventilstößel (7) und
- mit einer im Ventilgehäuse (2) beweglich geführten und mit dem Ventilstößel (7) verbundenen Betätigungswelle (8), wobei eine Bewegung der Betätigungswelle (8) auf den Ventilstößel (7) übertragbar ist, um diesen zwischen der Schließstellung und der Offenstellung zu bewegen,
**dadurch gekennzeichnet, dass** der Ventilstößel (7) in einem lösbar mit dem Ventilgehäuse (2) verbundenen Adapterkörper (9) beweglich gelagert ist.

2. Probenahmeventil (1) nach Anspruch 1, **dadurch gekennzeichnet, dass** der Ventilstößel (7) einen Stößelabschnitt (10) aufweist, der mit einem am Adapterkörper (9) ausgebildeten Ventilsitz (13) und/oder einer am Adapterkörper (9) ausgebildeten Dichtfläche (14) dichtend zusammenwirkt.

3. Probenahmeventil (1) nach Anspruch 1 oder 2, **dadurch gekennzeichnet, dass** der Adapterkörper (9) mit dem Ventilgehäuse (2) durch eine Rast-, Schraub-, Klemm-und/oder Steckverbindung, insbesondere gesicherte Steckverbindung (15), verbunden ist.

4. Probenahmeventil (1) nach einem der vorangehenden Ansprüche, **dadurch gekennzeichnet, dass** der Adapterkörper (9) gegenüber dem Ventilgehäuse (2) durch mindestens ein Dichtelement (16), insbesondere einen O-Ring, abgedichtet ist.

5. Probenahmeventil (1) nach einem der Ansprüche 2 bis 4, **dadurch gekennzeichnet, dass** der Stößelabschnitt (10) gegenüber dem Adapterkörper (9), insbesondere der Dichtfläche (14) oder den Ventilsitz (13), durch ein Dichtelement (12), insbesondere einen O-Ring, abgedichtet ist.

6. Probenahmeventil (1) nach einem der Ansprüche 2 bis 5, **dadurch gekennzeichnet, dass** der Stößelabschnitt (10) mindestens einen Ventilteller (11) aufweist.

7. Probenahmeventil (1) nach einem der vorangehenden Ansprüche, **dadurch gekennzeichnet, dass** der Adapterkörper (9) einen Anschluss (17) zur Verbindung mit der Rohrleitung aufweist.

8. Probenahmeventil (1) nach einem der vorangehenden Ansprüche, **dadurch gekennzeichnet, dass** der Adapterkörper (9) mit mindestens einer Entleerungsbohrung (18) versehen ist, die vorzugsweise in Strömungsrichtung hinter dem Ventilsitz (13) oder der Dichtfläche (14) liegt.

9. Probenahmeventil (1) nach einem der vorangehenden Ansprüche, **dadurch gekennzeichnet, dass** das mit dem Ventilstößel (7) verbundene Ende der Betätigungswelle (8) in Offenstellung in das Innere des Adapterkörpers eindringt.

10. Probenahmeventil (1) nach einem der vorangehenden Ansprüche, **dadurch gekennzeichnet, dass** der Adapterkörper (9), der Ventilstößel (7) und/oder die Betätigungswelle (8) koaxial zueinander angeordnet sind.

11. Probenahmeventil (1) nach einem der vorangehenden Ansprüche, **dadurch gekennzeichnet, dass** die Bewegung der Betätigungswelle (8) translatorisch und/oder rotatorisch ist.

12. Probenahmeventil (1) nach einem der vorangehenden Ansprüche, **dadurch gekennzeichnet, dass** die Bewegung des Ventilstößels (7) translatorisch und/oder rotatorisch ist.

13. Probenahmeventil (1) nach einem der vorangehenden Ansprüche, **dadurch gekennzeichnet, dass** die Betätigungswelle (8) als Spindel ausgebildet ist und insbesondere in einen im Ventilgehäuse (2) ausgebildeten Innengewinde (19) geführt ist.

14. Probenahmeventil (1) nach einem der vorangehenden Ansprüche, **dadurch gekennzeichnet, dass** die Betätigungswelle (8) und der Ventilstößel (7) lose miteinander verbunden sind und insbesondere in Flächenkontakt miteinander stehen.

15. Probenahmeventil (1) nach Anspruch 14, **dadurch gekennzeichnet, dass** die Betätigungswelle (8) eine Aufnahme für ein Ende des Ventilstößels (7) hat.

16. Probenahmeventil (1) nach einem der vorangehenden Ansprüche, **dadurch gekennzeichnet, dass** der Ventilstößel (7) in Richtung der Schließstellung mit Federkraft beaufschlagt ist.

17. Probenahmeventil (1) nach Anspruch 16, **dadurch gekennzeichnet, dass** eine Schraubenfeder (21) im Innern des Adapterkörpers (9) angeordnet ist, um die Federkraft auf den Ventilstößel (7) zu übertragen.

18. Probenahmeventil (1) nach einem der vorangehenden Ansprüche, **dadurch gekennzeichnet, dass** das Ventilgehäuse (2) T-förmig ausgebildet ist, wobei der Strömungskanal (5) am Ventilauslass (4) in einem Winkel von insbesondere 90° zum Strömungskanal (5) am Ventileinlass (3) angeordnet ist.

19. Probenahmeventil (1) nach einem der vorangehenden Ansprüche, **dadurch gekennzeichnet, dass** der Adapterkörper (9) im Ventilgehäuse (2) drehbar, insbesondere um 360°, angeordnet ist.

20. Probenahmeventil (1) nach einem der vorangehenden Ansprüche, **dadurch gekennzeichnet, dass** das Ablassrohr (6) relativ zum Ventilgehäuse (2) drehbar, insbesondere um 360°, angeordnet ist.

21. Probenahmeventil (1) nach Anspruch 20, **dadurch gekennzeichnet, dass** das Ablassrohr (6) mit dem Ventilgehäuse (2) über einen Klemmring (22) und eine Druckschraube (23) verbunden ist.

22. Probenahmeventil (1) nach einem der vorangehenden Ansprüche, **dadurch gekennzeichnet, dass** das Ventilgehäuse (2), der Adapterkörper (9), der Ventilstößel (7) und/oder die Betätigungswelle (8) aus Rotguss und/oder Edelstahl gefertigt sind.

23. Ventileinsatz (24) zum lösbaren Verbinden mit einem Ventilgehäuse (2), insbesondere einem Ventilgehäuse (2) eines Probenahmeventils (1),
- mit einem im Ventilgehäuse (2) hin- und herbewegbaren, einen Strömungskanal (5) im Ventilgehäuse (2) wahlweise in einer Schließstellung verschließenden und in einer Offenstellung freigebenden Ventilstößel (7) und
- mit einem Adapterkörper (9), in dem der Ventilstößel (7) beweglich gelagert ist.

24. Ventileinsatz (24) nach Anspruch 23, **dadurch gekennzeichnet, dass** der Ventilstößel (7) einen Stößelabschnitt (10) aufweist, der mit einem am Adapterkörper (9) ausgebildeten Ventilsitz (13) und/oder einer am Adapterkörper (9) ausgebildeten Dichtfläche (14) dichtend zusammenwirkt.

25. Ventileinsatz (24) nach Anspruch 23 oder 24, **dadurch gekennzeichnet, dass** der Adapterkörper (9) mit dem Ventilgehäuse (2) durch eine Rast-, Schraub-, Klemm-und/oder Steckverbindung, insbesondere gesicherte Steckverbindung (15), verbindbar ist.

26. Ventileinsatz (24) nach einem der Ansprüche 23 bis 25, **dadurch gekennzeichnet, dass** der Adapterkörper (9) gegenüber dem Ventilgehäuse (2) durch mindestens ein Dichtelement (16), insbesondere einen O-Ring, abdichtbar ist.

27. Ventileinsatz (24) nach einem der Ansprüche 24 bis 26, **dadurch gekennzeichnet, dass** der Stößelabschnitt (10) gegenüber dem Adapterkörper (9), insbesondere der Dichtfläche (14) oder den Ventilsitz (13), durch ein Dichtelement (12), insbesondere einen O-Ring, abdichtbar ist.

28. Ventileinsatz (24) nach einem der Ansprüche 24 bis 27, **dadurch gekennzeichnet, dass** der Stößelabschnitt (10) mindestens einen Ventilteller (11) aufweist.

29. Ventileinsatz (24) nach einem der Ansprüche 23 bis 28, **dadurch gekennzeichnet, dass** der Adapterkörper (9) einen Anschluss (17) zur Verbindung mit der Rohrleitung aufweist.

30. Ventileinsatz (24) nach einem der Ansprüche 23 bis 29, **dadurch gekennzeichnet, dass** der Adapterkörper (9) mit mindestens einer Entleerungsbohrung (18) versehen ist, die vorzugsweise in Strömungsrichtung hinter dem Ventilsitz (13) oder der Dichtfläche (14) liegt.

31. Ventileinsatz (24) nach einem der Ansprüche 23 bis 30, **dadurch gekennzeichnet, dass** der Adapterkörper (9) und der Ventilstößel (7) koaxial zueinander angeordnet sind.

32. Ventileinsatz (24) nach einem der Ansprüche 23 bis 31, **dadurch gekennzeichnet, dass** die Bewegung des Ventilstößels (7) translatorisch und/oder rotatorisch ist.

33. Ventileinsatz (24) nach einem der Ansprüche 23 bis 32, **dadurch gekennzeichnet, dass** der Ventilstößel (7) in Richtung der Schließstellung mit Federkraft beaufschlag ist.

34. Ventileinsatz (24) nach Anspruch 33, **dadurch gekennzeichnet, dass** eine Schraubenfeder (21) im Innern des Adapterkörpers (9) angeordnet ist, um die Federkraft auf den Ventilstößel (7) zu übertragen.

35. Ventileinsatz (24) nach einem der Ansprüche 23 bis 34, **dadurch gekennzeichnet, dass** der Adapterkörper (9) und/oder der Ventilstößel (7) aus Rotguss und/oder Edelstahl gefertigt ist.

## Claims

1. A sample valve (1) for the sterile taking of samples from a container or a pipeline system
- comprising a valve housing (2) with a valve inlet (3), a valve outlet (4) and a flow duct (5) connecting them, wherein the valve inlet (3) can be fluidically connected to a pipeline conducting a removable medium and wherein the valve outlet (4) can be fluidically connected to a discharge tube (6),
- comprising a valve tappet (7), which can be moved back and forth in the valve housing (2) and which selectively seals the flow duct (5) in a closed position and releases the flow duct (5) in an open position and
- comprising an actuating shaft (8), which is movably guided in the valve housing (2) and which is connected to the valve tappet (7), wherein a movement of the actuating shaft (8) can be transferred to the valve tappet (7) so as to move said valve tappet (7) between the closed position and the open position,
**characterized in that** the valve tappet (7) is displaceably supported in an adapter body (9), which is removably connected to the valve housing (2).

2. The sample valve (1) according to claim 1, **characterized in that** the valve tappet (7) comprises a tappet section (10) which interacts in a sealing manner with a valve seat (13) embodied on the adapter body (9) and/or with a sealing surface (14) embodied on the adapter body (9).

3. The sample valve (1) according to claim 1 or 2, **characterized in that** the adapter body (9) is connected to the valve housing (2) by means of a locking connection, screw connection, clamping connection and/or plug-in connection, in particular by means of a secured plug-in connection (15).

4. The sample valve (1) according to one of the preceding claims, **characterized in that** the adapter body (9) is sealed by means of at least one sealing element (16), in particular an 0-ring, against the valve housing (2).

5. The sample valve (1) according to one of claims 2 to 4, **characterized in that** the tappet section (10) is sealed by means of a sealing element (12), in particular an O-ring, against the adapter body (9), in particular the sealing surface (14) or the valve seat (13).

6. The sample valve (1) according to one of claims 2 to 5, **characterized in that** the tappet section (10) comprises at least one valve disk (11).

7. The sample valve (1) according to one of the preceding claims, **characterized in that** the adapter body (9) comprises a connection (17) for connecting to the pipeline.

8. The sample valve (1) according to one of the preceding claims, **characterized in that** the adapter body (9) is provided with at least one discharge bore (18), which is preferably located in flow direction downstream from the valve seat (13) or the sealing surface (14).

9. The sample valve (1) according to one of the preceding claims, **characterized in that** the end of the actuating shaft (8) connected to the valve tappet (7) penetrates the interior of the adapter body in the open position.

10. The sample valve (1) according to one of the preceding claims, **characterized in that** the adapter body (9), the valve tappet (7) and/or the actuating shaft (8) are arranged so as to be coaxial to one another.

11. The sample valve (1) according to one of the preceding claims, **characterized in that** the movement of the actuating shaft (8) is translatory and/or rotatory.

12. The sample valve (1) according to one of the preceding claims, **characterized in that** the movement of the valve tappet (7) is translatory and/or rotatory.

13. The sample valve (1) according to one of the preceding claims, **characterized in that** the actuating shaft (8) is embodied as a spindle and is guided in particular in an internal thread (19) embodied in the valve housing (2).

14. The sample valve (1) according to one of the preceding claims, **characterized in that** the actuating shaft (8) and the valve tappet (7) are loosely connected to one another and are in particular in surface contact with one another.

15. The sample valve (1) according to claim 14, **characterized in that** the actuating shaft (8) has an accommodation for an end of the valve tappet (7).

16. The sample valve (1) according to one of the preceding claims, **characterized in that** the valve tappet (7) is biased by spring force in the direction of the closed position.

17. The sample valve (1) according to claim 16, **characterized in that** a helical spring (21) is arranged in the interior of the adapter body (9) so as to transfer the spring force to the valve tappet (7).

18. The sample valve (1) according to one of the preceding claims, **characterized in that** the valve housing (2) is embodied so as to be T-shaped, wherein the flow duct (5) is arranged on the valve outlet (4) at an angle of in particular 90° to the flow channel (5) on the valve inlet (3).

19. The sample valve (1) according to one of the preceding claims, **characterized in that** the adapter body (9) is arranged in the valve housing (2) so as to be rotatable, in particular by 360°.

20. The sample valve (1) according to one of the preceding claims, **characterized in that** the discharge tube (6) is arranged relative to the valve housing (2) so as to be rotatable, in particular by 360°.

21. The sample valve (1) according to claim 20, **characterized in that** the discharge tube (6) is connected to the valve housing (2) via a clamping ring (22) and a pressing screw (23).

22. The sample valve (1) according to one of the preceding claims, **characterized in that** the valve housing (2), the adapter body (9), the valve tappet (7) and/or the actuating shaft (8) are made of red brass and/or stainless steel.

23. A valve insert (24) for removably connecting to a valve housing (2), in particular a valve housing (2) of a sample valve (1),
- comprising a valve tappet (7), which can be moved back and forth in the valve housing (2) and which selectively seals a flow duct (5) in a closed position and releases the flow duct (5) in an open position and
- comprising an adapter body (9), in which the valve tappet (7) is movably supported.

24. The valve insert (24) according to claim 23, **characterized in that** the valve tappet (7) comprises a tappet section (10), which interacts in a sealing manner with a valve seat (13) embodied on the adapter body (9) and/or with a sealing surface (14) embodied on the adapter body (9).

25. The valve insert (24) according to claim 23 or 24, **characterized in that** the adapter body (9) is connected to the valve housing (2) by means of a locking connection, screw connection, clamping connection and/or plug-in connection, in particular by means of a secured plug-in connection (15).

26. The valve insert (24) according to one of claims 23 to 25, **characterized in that** the adapter body (9) is sealed by means of at least one sealing element (16), in particular an 0-ring, against the valve housing (2).

27. The valve insert (24) according to one of claims 24 to 26, **characterized in that** the tappet section (10) can be sealed by means of a sealing element (12), in particular an O-ring, against the adapter body (9), in particular the sealing surface (14) or the valve seat (13).

28. The valve insert (24) according to one of claims 24 to 27, **characterized in that** the tappet section (10) comprises at least one valve disk (11).

29. The valve insert (24) according to one of claims 23 to 28, **characterized in that** the adapter body (9) comprises a connection (17) for connecting to the pipeline.

30. The valve insert (24) according to one of claims 23 to 29, **characterized in that** the adapter body (9) is provided with at least one discharge bore (18), which is preferably located in flow direction downstream from the valve seat (13) or the sealing surface (14).

31. The valve insert (24) according to one of claims 23 to 30, **characterized in that** the adapter body (9) and the valve tappet (7) are arranged so as to be coaxial to one another.

32. The valve insert (24) according to one of claims 23 to 31, **characterized in that** the movement of the valve tappet (7) is translatory and/or rotatory.

33. The valve insert (24) according to one of claims 2 to 32, **characterized in that** the valve tappet (7) is biased by a spring force in the direction of the closed position.

34. The valve insert (24) according to claim 33, **characterized in that** a helical spring (21) is arranged in the interior of the adapter body (9) so as to transfer the spring force to the valve tappet (7).

35. The valve insert (24) according to one of claims 23 to 34, **characterized in that** the adapter body (9) and/or the valve tappet (7) are made of red brass and/or stainless steel.

## Revendications

1. Vanne de prélèvement d'échantillons (1) destinée au prélèvement stérile d'échantillons d'un récipient ou d'un système de conduite
- comprenant un carter de vanne (2) avec une entrée de vanne (3), une sortie de vanne (4) et un canal d'écoulement (5) reliant ces dernières, l'entrée de vanne (3) étant en communication de fluide avec une conduite guidant un fluide pouvant être prélevé et la sortie de vanne (4) pouvant être en communication de fluide avec un tuyau d'évacuation (6)
- comprenant un poussoir (7) mobile en va-et-vient dans le carter de vanne (2), au choix fermant dans une position de fermeture et libérant dans une position d'ouverture le canal d'écoulement (5) et
- comprenant un arbre d'actionnement (8) guidé mobilement dans le carter de vanne (2) et relié au poussoir (7), un déplacement de l'arbre d'actionnement (8) pouvant être transmis au poussoir (7) pour le déplacer entre la position d'ouverture et la position de fermeture, **caractérisée en ce que** le poussoir (7) est logé de manière mobile dans un corps d'adaptateur (9) relié de manière mobile au carter de vanne (2).

2. Vanne de prélèvement d'échantillons (1) selon la revendication 1, **caractérisé en ce que** le poussoir (7) présente une section de poussoir (10) qui coopère de manière étanche avec un siège de vanne (13) ménagé sur le corps d'adaptateur (9) et/ou une surface d'étanchéité (14) ménagée sur le corps d'adaptateur (9).

3. Vanne de prélèvement d'échantillons (1) selon la revendication 1 ou 2, **caractérisé en ce que** le corps d'adaptateur (9) est relié au carter de vanne (2) par une connexion d'encliquetage, de vis, de serrage et/ou d'emboitement en particulier une connexion par fiche sécurisée (15).

4. Vanne de prélèvement d'échantillons (1) selon l'une des revendications précédentes, **caractérisé en ce que** le corps d'adaptateur (9) est rendu étanche par rapport au carter de vanne (2) par au moins un élément d'étanchéité (16), en particulier un joint torique.

5. Vanne de prélèvement d'échantillons (1) selon l'une des revendications 2 à 4, **caractérisée en ce que** la section de poussoir (10) est rendue étanche par rapport au corps d'adaptateur (9), en particulier par rapport à la surface étanche (14) ou le siège de vanne (13), par un élément d'étanchéité (12), en particulier un joint torique.

6. Vanne de prélèvement d'échantillons (1) selon l'une des revendications 2 à 5, **caractérisée en ce que** la section de poussoir (10) présente au moins un disque de vanne (11).

7. Vanne de prélèvement d'échantillons (1) selon l'une des revendications précédentes, **caractérisée en ce que** le corps d'adaptateur (9) présente un raccordement (17) pour la connexion à la conduite.

8. Vanne de prélèvement d'échantillons (1) selon l'une des revendications précédentes, **caractérisée en ce que** le corps d'adaptateur (9) est muni au moins d'un perçage de vidage (18) qui se trouve de préférence dans le sens d'écoulement derrière le siège de vanne (13) ou la surface d'étanchéité (14).

9. Vanne de prélèvement d'échantillons (1) selon l'une des revendications précédentes, **caractérisée en ce que** l'extrémité de l'arbre d'actionnement (8) reliée au poussoir (7) pénètre en position d'ouverture dans l'intérieur du corps d'adaptateur.

10. Vanne de prélèvement d'échantillons (1) selon l'une des revendications précédentes, **caractérisée en ce que** le corps d'adaptateur (9), le poussoir (7) et/ou l'arbre d'actionnement (8) sont disposés coaxialement entre eux.

11. Vanne de prélèvement d'échantillons (1) selon l'une des revendications précédentes, **caractérisée en ce que** le mouvement de l'arbre d'actionnement (8) est une rotation et/ou une translation.

12. Vanne de prélèvement d'échantillons (1) selon l'une des revendications précédentes, **caractérisée en ce que** le mouvement du poussoir (7) est une rotation et/ou une translation.

13. Vanne de prélèvement d'échantillons (1) selon l'une des revendications précédentes, **caractérisée en ce que** l'arbre d'actionnement (8) est réalisé comme une broche et est en particulier guidée dans un filetage interne (19) ménagé dans le carter de vanne (2).

14. Vanne de prélèvement d'échantillons (1) selon l'une des revendications précédentes, **caractérisée en ce que** l'arbre d'actionnement (8) et le poussoir (7) sont reliés de manière lâche et sont en particulier en contact de surface entre eux.

15. Vanne de prélèvement d'échantillons (1) selon la revendication 14, **caractérisée en ce que** l'arbre d'actionnement (8) présente un logement pour une extrémité du poussoir (7).

16. Vanne de prélèvement d'échantillons (1) selon l'une des revendications précédentes, **caractérisée en ce que** le poussoir (7) subit une force de ressort dans la direction de la position de fermeture.

17. Vanne de prélèvement d'échantillons (1) selon la revendication 14, **caractérisée en ce qu'**un ressort hélicoïdal (21) est disposé à l'intérieur du corps d'adaptateur (9) pour transmettre la force de ressort au poussoir (7).

18. Vanne de prélèvement d'échantillons (1) selon l'une des revendications précédentes, **caractérisée en ce que** le carter de vanne (2) présente la forme d'un T, le canal d'écoulement (5) étant disposé sur la sortie de vanne (4) en formant un angle en particulier de 90° par rapport au canal d'écoulement (5) sur l'entrée de vanne (3).

19. Vanne de prélèvement d'échantillons (1) selon l'une des revendications précédentes, **caractérisée en ce que** le corps d'adaptateur (9) est disposé dans le carter de vanne (2) de manière rotative, en particulier sur 360°.

20. Vanne de prélèvement d'échantillons (1) selon l'une des revendications précédentes, **caractérisée en ce que** le tuyau d'évacuation (6) est disposé dans le carter de vanne (2) de manière rotative, en particulier sur 360°.

21. Vanne de prélèvement d'échantillons (1) selon la revendication 20, **caractérisée en ce que** le tuyau d'évacuation (6) est relié au carter de vanne (2) par un anneau de serrage (22) et une vis de pression (23).

22. Vanne de prélèvement d'échantillons (1) selon l'une des revendications précédentes, **caractérisée en ce que** le carter de vanne (2), le corps adaptateur (9), le poussoir (7) et/ou l'arbre d'actionnement (8) sont fabriqués en laiton rouge ou en acier inoxydable.

23. Garniture de vanne (24) pour la connexion amovible avec un carter de vanne (2), en particulier un carter de vanne (2) d'une vanne de prélèvement d'échantillons (1),
- comprenant avec un poussoir (7) mobile en va-et-vient dans le carter de vanne (2), au choix fermant dans une position de fermeture et libérant dans une position d'ouverture un canal d'écoulement (5) et
- comprenant avec un corps d'adaptateur (9), dans lequel est logé le poussoir (7) de manière mobile.

24. Garniture de vanne (24) selon la revendication 23, **caractérisée en ce que** le poussoir (7) présente une section de poussoir (10) qui coopère de manière étanche avec un siège de vanne (13) ménagé sur le corps d'adaptateur (9) et/ou une surface d'étanchéité (14) ménagée sur le corps d'adaptateur (9).

25. Garniture de vanne (24) selon la revendication 23 ou 24, **caractérisée en ce que** le corps d'adaptateur (9) est relié au carter de vanne (2) par une connexion d'encliquetage, de vis, de serrage et/ou d'emboitement en particulier une connexion par fiche sécurisée (15).

26. Garniture de vanne (24) selon l'une des revendications 23 à 24, **caractérisée en ce que** le corps d'adaptateur (9) est rendu étanche par rapport au carter de vanne (2) par au moins un élément d'étanchéité (16), en particulier un joint torique.

27. Garniture de vanne (24) selon l'une des revendications 24 à 26, **caractérisée en ce que** la section de poussoir (10) est rendue étanche par rapport au corps d'adaptateur (9), en particulier par rapport à la surface étanche (14) ou le siège de vanne (13), par un élément d'étanchéité (12), en particulier un joint torique.

28. Garniture de vanne (24) selon l'une des revendications 24 à 27, **caractérisée en ce que** la section de poussoir (10) présente au moins un disque de vanne (11).

29. Garniture de vanne (24) selon l'une des revendications 23 à 28, **caractérisée en ce que** le corps d'adaptateur (9) présente un raccordement (17) pour la connexion à la conduite.

30. Garniture de vanne (24) selon l'une des revendications 23 à 29, **caractérisée en ce que** le corps d'adaptateur (9) est muni au moins d'un perçage de vidage (18) qui se trouve de préférence dans le sens d'écoulement derrière le siège de vanne (13) ou la surface d'étanchéité (14).

31. Garniture de vanne (24) selon l'une des revendications 23 à 30, **caractérisée en ce que** le corps d'adaptateur (9) et le poussoir (7) sont disposés coaxialement entre eux.

32. Garniture de vanne (24) selon l'une des revendications 23 à 31, **caractérisée en ce que** le mouvement du poussoir (7) est une rotation et/ou une translation.

33. Garniture de vanne (24) selon l'une des revendications 23 à 32, **caractérisée en ce que** le poussoir (7) subit une force de ressort dans la direction de la position de fermeture.

34. Garniture de vanne (24) selon la revendication 33, **caractérisée en ce qu'**un ressort hélicoïdal (21) est disposé à l'intérieur du corps d'adaptateur (9) pour transmettre la force de ressort au poussoir (7).

35. Garniture de vanne (24) selon l'une des revendications 23 à 34, **caractérisée en ce que** le corps adaptateur (9) et/ou le poussoir (7) sont fabriqués en laiton rouge ou en acier inoxydable.
